# NEW EUROPEAN PATENT SPECIFICATION

(11) **EP 2 012 765 B2**
(45) Date of publication and mention of the opposition decision: **11.04.2018**
(45) Mention of the grant of the patent: 25.03.2015
(21) Application number: 07735699.6
(22) Date of filing: 27.04.2007
(51) Int. Cl.: A61K 31/137, A61K 31/167, A61K 31/485, A61K 47/10, A61K 47/12, A61K 47/20, A61P 11/02, A61P 11/10

(54) **LIQUID COMPOSITIONS COMPRISING PHENYLEPHRINE AND ACETAMINOPHEN AND THEIR USE FOR THE TREATMENT OF RESPIRATORY ILLNESS**
FLÜSSIGE VERBINDUNGEN ENTHALTEND PHENYLEPHRINE UND ACTETAMINOPHEN SOWIE DEREN VERWENDUNG ZUR BEHANDLUNG VON ATEMWEGSERKRANKUNGEN
COMPOSITIONS LIQUIDES COMPRENANT DU PHENYLEPHRINE ET DE L'ACETAMINOPHEN ET L'UTILISATION POUR LE TRAITEMENT D'UNE MALADIE RESPIRATOIRE

(30) Priority: 28.04.2006 US 413766
(43) Date of publication of application: 14.01.2009
(73) Proprietor: The Procter & Gamble Company, Cincinnati, OH 45202 (US)
(72) Inventor: MARTIN, Kelly, Lee, Cincinnati, Ohio 45227 (US); CRISS, Susan, Elaine, Maineville, Ohio 45039 (US); GLEDHILL, Douglas, William, Cincinnati, Ohio 45212 (US); ONONYE, Aloysius, Ike, Loveland, Ohio 45140 (US)
(74) Representative: Töpert, Verena Clarita
(86) International application number: PCT/IB2007/051583
(87) International publication number: WO 2007/125501

(56) References cited:
- WO-A-96/14828
- WO-A-03/047502
- WO-A-2007/098128
- WO-A1-2004/066978
- US-A- 3 480 185
- US-A1- 2007 098 785
- DFAS GUPTA V ET AL: "CHEMICAL STABILITIES OF LIGNOCAINE HYDROCHLORIDE AND PHENYLEPHRINE HYDROCHLORIDE IN AQUEOUS SOLUTION" JOURNAL OF CLINICAL AND HOSPITAL PHARMACY, BLACKWELL SCIENTIFIC PUBLICATIONS, LONDON, GB, vol. 11, no. 6, 1986, pages 449-452, XP002056657 ISSN: 0143-3180
- ANONYMOUS: "Alka Seltzer Plus Cold & Cough Liquid" INTERNET, [Online] 3 November 2006 (2006-11-03), XP002452249 Retrieved from the Internet: URL:HTTP://WWW.ALKASELTZER.COM/ASP/PRODUCT S/COLCOUGH_LIQUID.HTML> [retrieved on 2007-09-20]

## Description

### FIELD OF THE INVENTION

The present invention relates to liquid compositions useful for treatment of respiratory illness such as cold, flu, allergies, sinusitis, and rhinitis. More particularly, the invention relates to liquid compositions comprising phenylephrine, its free and addition salt forms and mixtures thereof, and acetaminophen, wherein the compositions have a defined pH.

### BACKGROUND OF THE INVENTION

Respiratory illness encompasses a broad range of ailments, including viral infections such as cold and flu, as well as allergies, sinusitis, rhinitis, and the like. Respiratory illness may present as any of a variety of symptoms, such as runny nose, nasal or chest congestion, cough, sneezing, pressure, headache, aches, fever or sore throat. Pharmaceutical actives typically used to treat these symptoms generally fall into one of the following pharmaceutical classifications: antihistamines, decongestants, antitussives, expectorants, demulcents, anesthetics, analgesics, antipyretic and anti-inflammatory agents. The products for treating respiratory symptoms associated with respiratory illness are manufactured in a number of product forms, the most common being liquid syrups and elixirs for swallowing, mouth and throat drops and lozenges, tablets, caplets, capsules, and liquid-filled capsules and lozenges, effervescent tablets, and dry dissolvable powders, as well as inhalants and topical creams and lotions that release volatile agents that are inhaled through the nose into the respiratory tract. The oral compositions are typically swallowed immediately, or slowly dissolved in the mouth.

Products for relief of multiple symptoms may include various pharmaceutical actives such as pseudoephedrine, phenylephrine, and phenylpropanolamine (decongestants), guaifenesin (an expectorant), chlorpheniramine, diphenhydramine and doxylamine (antihistamines), dextromethorphan (cough suppressant), acetaminophen, ibuprofen, and aspirin (analgesics). Because these actives have different properties and stabilities, it is a challenge to formulate overall compositions containing actives wherein the actives are all stable and effective. In particular, the stability of certain pharmaceutical actives has been an on-going problem, especially when formulated in combination with other actives. Often, for example, liquid solutions discolor or one or more actives precipitates out of solution or is degraded. To illustrate, wherein phenylephrine is desired as a pharmaceutical active, one of the common problems associated with the formulation and use of phenylephrine is degradation. Phenylephrine may degrade in the presence of oxygen, aldehydes, certain acids including citric acid, and metals. The degradation or phenylephrine, even in solid dose forms, has also been reported.

Patent application WO 2004/066978 discloses a dissolvable powder pharmaceutical composition comprising 0.2% by weight of phenylephrine, 10 % by weight of acetaminophen and 10.5% by weight of citric acid.

Thus, there is an ongoing need for stable, effective compositions useful for the treatment of respiratory illness and associated symptoms.

### SUMMARY OF THE INVENTION

The present invention is directed to liquid compositions comprising phenylephrine, its free and addition salt forms and mixtures thereof, and acetaminophen. The compositions have a pH of from 6.5 to 7.5. The compositions can be substantially free of aldehydes for further enhanced stability. The compositions can be in the form of, for example, liquids, elixirs, liquid-filled capsules, liquid-filled lozenges, dissolvable compositions, and inhalants, but are preferably orally administrable liquid forms. The invention is also directed to a method of stabilizing phenylephrine. The invention is further directed to methods of treating respiratory illness and symptoms thereof comprising orally administering a composition as described herein.

These and other aspects of the present invention are described in further detail herein.

### DETAILED DESCRIPTION OF THE INVENTION

All weights, measurements and concentrations herein are measured at 25°C on the composition in its entirety, unless otherwise specified.

All percentages and ratios are calculated by weight unless otherwise indicated. All percentages and ratios are calculated based on the total composition unless otherwise indicated.

Referenced herein may be trade names for components including various ingredients utilized in the present invention. The inventors herein do not intend to be limited by materials under a certain trade name. Equivalent materials (e.g., those obtained from a different source under a different name or reference number) to those referenced by trade name may be substituted and utilized in the descriptions herein.

In description of the invention, various embodiments or individual features are disclosed. As will be apparent to the ordinarily skilled practitioner, all combinations of such embodiments and features are possible and can result in preferred executions of the present invention.

Compositions of the Present Invention The present compositions are defined herein in a number of embodiments, all relating to the discoveries made by the present inventors. In particular, the inventors have discovered that the compositions of the present invention are made stable through combination of phenylephrine and acetaminophen in a formulation at a defined pH. Additional stability benefit can be achieved if the compositions are substantially free of aldehydes.

### Phenylephrine and Acetaminophen

In one embodiment, the liquid compositions of the present invention comprise phenylephrine; its free and addition salt forms, and mixtures thereof, and acetaminophen.

Illustrative salts of phenylephrine include phenylephrine hydrochloride and phenylephrine hydrobromide. For simplicity herein, phenylephrine and its free and addition salt forms and mixtures thereof may be collectively referred to as "phenylephrine".

In one embodiment, the compositions of the present invention may comprise an amount of phenylephrine in the range of 0.0001 mg to 20 mg of phenylephrine, alternatively from 0.01 to 15 mg, and alternatively from 5 mg to 10 mg of phenylephrine, all per dose or the composition. By way of, example, an embodiment of the present invention may comprise 10 mg of phenylephrine, per dose. Another embodiment of the present invention may comprise 5 mg of phenylephrine, per dose.

Alternatively or additionally, in an embodiment of the present invention, the compositions of the present invention may comprise an amount of phenylephrine in the range of from 0.0001% to 1%, alternatively from 0.001 % to 0.5%, and alternatively from 0.01 % to 0.25%, all by weight of the composition.

In one embodiment, the compositions of the present invention may comprise an amount of acetaminophen in the range of 150 to 1,000 mg, alternatively from 200 to 750 mg, and alternatively from 500 to 650 mg of acetaminophen, all per dose of the composition.

Alternatively or additionally, in an embodiment of the present invention. the compositions of the invention may comprise amount of acetaminophen in the range of from 0.01% to 10%, alternatively from 1% to 7%, and alternatively from 2 % to 5%, all by weight of the composition.

### Additional Pharmaceutical Actives

The compositions of the present invention can also comprise an additional pharmaceutical active. Pharmaceutical actives and pharmaceutically acceptable forms thereof are readily known to the ordinarily skilled artisan and, as such, the actives arc not bound by the descriptions provided herein. As illustrative examples, pharmaceutical actives may include antitussives, antihistamines, non-sedating antihistamines, decongestants, expectorants, analgesic, antipyretic anti-inflammatory agents, local anesthetics, anti-inflammatory agents, demulcent, and mixtures thereof.

By way of further illustration, specilic additional pharmaceutical actives include all pharmaceutically acceptable forms of dextromethorphan, ephedrine, pseudoephedrine, phenylpropanolamine, ibuprofen, aspirin, ketoprofen, guaifenesin, anthroxyl, bromthexine, diphenhydramine, chlorphenirantine, doxylantine, triprolidine, clemastine, pyrilamine, promethazine, cetirizine, loratidine, oxycodone, hydrocodone, naproxen, brompheniramine, carbinoxamine, caffeine, benzonatate, pheniramine, fentanyl, azatedine, desloratadine, carbamazepine, buprenorphine, hydromorphone, indomethacin, oxymorphone, phenol, codeine, mesalamine, dichlophenac, sulindac, beclomethaxone, meloxicam, fenoproten, mometasone, menthol, benzocaine, dipyridamole, methscopolamine, the free and the addition salt forms thereof, and mixtures thereof.

In one embodiment additional pharmaceutical actives may include dextromethorphan, doxylantine, and guaifenesin.

In one embodiment, the compositions of the present invention may comprise an amount of additional pharmaceutical active in the range of zero (0) mg to 1,000 mg of each of at least one additional pharmaceutical active, alternatively from 2.5 mg to 750 mg, and alternatively prom 5 mg to 500 mg of each of at least one additional pharmaceutical active, all per dose of the composition.

In one embodiment, the compositions of the present invention may comprise an amount of additional pharmaceutical active in the range of 0% to 15%, alternatively 0.0001% to 10%, alternatively from 0.001% to 7%, and alternatively from 0.01 % to 5%, all by weight of the composition.

### pH

The present inventors have herein discovered that phentylephrine, present in the liquid compositions herein may achieve enhanced stability wherein the composition has a pH of from 6.5 to 7.5, and alternatively from 6.75 to 7.25.

These results are surprising in light of published literature which cites acidic pH as favorable or even necessary for stability ot phenylepluine.

Regardless of the actual mechanism(s), the present inventors find that compositions having the pH as defined herein, assist greatly in the stabilization of phenylephrine in phenylephrine-acetaminophen combinations. However it has also been noted by the inventors that certain pharmaceutical actives can react negatively with certain organic buffers such as citrate buffers. Therefore, wherein certain buffers are used, the buffer should be used at low levels, using only enough to achieve and maintain the desired pH.

Therefore, for example, the present compositions may comprise one or more buffers in order to reach, and maintain, the presently defined pH. pH can be adjusted to and maintained within the requisite range by known and conventional methods. Buffers, as used herein, are substances added to a composition to modify and maintain the pH of the composition. Organic as well as inorganic edible buffers, such as phosphate buffers may be used to adjust the pH of the liquid compositions herein.

### Substantially Free of Aldehydes

The compositions of the present invention may also be substantially free of aldehydes. As used herein, substantially free of aldehydes means that the composition comprise less than 0.1%, alternatively less than 0.05%, alternatively less than 0.01%, and alternatively less than 0.001% total aldehydes, (i.e. compounds containing at least one aldehydic moiety), all by weight of the composition. As the inventors have discovered, formulating the compositions of the present invention to be substantially free of aldehydes upon manufacture can compensate for the potential for formation of some amount of aldehyde in the composition during storage conditions.

Aldehydes are compounds that are well known to the ordinarily skilled artisan.
Flavors are well known for use in health products for improving consumer acceptance, and many such flavors are aldehydic in structure. For example, characterizing compounds for cherry flavors include benzaldehyde and p-tolyl aldehyde. However, the inventors have found that these same flavors also often cause degradation of the phenylephrine used herein.

The present inventors have found that substantial removal of the aldehydes, as defined herein, greatly stabilizes the resulting composition. Thus, given the desire to provide compositions that are aesthetically acceptable, and stable, the present invention further provides optional alternatives to flavors and aromas typically containing significant levels of aldehydes. Such alternatives are herein referenced as non-aldehydic aesthetic agents.

To illustrate, the inventors have discovered that typical flavors and aromas may be substituted with non-aldehydic aesthetic agents such as flavor components which are selected from the group consisting of esters, ketones and alcohols, and also sweeteners, and mixtures thereof, in order to formulate flavors that smell and taste like cherry or other desired flavors.

As further examples, the present compositions may comprise a non-aldehydic aesthetic agent such as an ester selected from the group consisting of ethyl butyrate, benzyl acetate, benzyl butyrate, allyl isovalerate, allyl caproate, ethyl-2-methyl butyrate, ethyl methyl phenyl glycidate, and mixtures thereof. Utilizing these fruity esters can readily generate flavors similar to cherry and berry flavors.

The body of the flavor may also be important to make it take on character and endure. The use of ketones such as ionones are useful for this purpose. To illustrate, oxanone (4-(p-hydroxyphenyl)-2-butanone, raspberry ketone) along with trace amounts of ionones can provide body to the flavor.

As a further example, compounds such as cis-3-hexenol and trans-2-hexenyl acetate may add to the flavor. Furaneol and maltol may add a candy-like nuance.

In addition, the compositions of the present invention may optionally comprise low-aldehyde juice concentrates, for example including peach, citrus and apricot, as flavoring agents.

The compositions of the present invention may optionally contain from 0.0001% to 5%, alternatively from 0.01% to 2%, and alternatively from 0.025% to 1.5% of non-aldehydic aesthetic agents, all by weight of the composition.

Other Optional Components of the Present Compositions Any or all components typically associated with respiratory illness and symptom treatment products can be used as required or as optional components herein. For example, exemplary components are disclosed in US Patent No. 5,196,436.

### Sweeteners

The present liquid compositions may optionally comprise a sugar and/or other sweetener to provide sweetness and taste masking of pharmaceutical active(s) as well as to provide some body and thickness. Sucrose, or table sugar, often in liquid form, may be used. However, sucrose can hydrolyze to its constituent sugars, namely glucose and fructose. Glucose is an aldehyde, and therefore may be less desirable for use herein. However, the present inventors discover herein that the effect of glucose on phenylephrine is less than that of traditional aldehyde-containing flavors and aromas. Nonetheless, improved stability can be achieved when low levels of sugar are used, in addition to inclusion of a non-aldehydic aesthetic agent if an aesthetic agent is used, such that the composition remains substantially free of aldehydes as described herein. Relatively hightly pure grades of sugars, having undergone less hydrolysis to monosaccharides, may assist in lowering levels of aldehydes as well. Corn syrup, including high fructose grades, can also be used, through is less desirable because corn syrup contains aldehydes.

For example, the compositions of the present invention can contain sugar, such as sucrose, in a liquid solution in the range of from 10% to 70% sugar solution by weight of the composition, and alternatively from 15% to 60% sugar solution by weight of the composition, wherein the sugar solution can comprises from 50% to 70% sugar by weight of the sugar solution.

Alternatively, or additionally if greater sweetening is desired, sugar alcohols such as glycerin, sorbitol, maltitol, and mannitol can be used to provide sweetness and body. If such sugar alcohol solutions are used, they can be used in a range of from % to 30% sugar alcohol solution by weight of the composition, and alternatively from 10% to 25% sugar alcohol solution by weight of the composition, wherein the sugar alcohol solution may comprise from 60% to 100% sugar alcohol by weight of the sugar alcohol solution. For example, a 70% by weight sugar alcohol solution can be used at 20% by weight of the composition, resulting in 14% sugar alcohol by weight of the composition.

Sweetness levels can also be supplemented with the use of an artificial sweetener. Examples of artificial sweeteners are selected from sodium saccharin, acesulfame potassium, sucralose, aspartame, monoammonium glycyrrhizinate, neohesperidin dihydrochalcone, thaumatin, neotame, cyclamates, and mixtures thereof. Generally, such artificial sweeteners are solids when used in sweetening compositions such as those of the present invention.

Wherein an artificial sweetener is utilized in the present inventive compositions, the compositions may comprise from 0.0001% to 5% artificial sweetener, alternatively from 0.0425% to 3.5%o artificial sweetener, and alternatively from 0.05% to 2.0% artificial sweetener, all by weight of the composition.

### Solvents

The present liquid components typically comprise a solvent. In one embodiment, the solvent is water-soluble or water miscible. As used herein, "solvent" means a substance used to dissolve phenylephrine, other pharmaceutical active(s), and other components of the composition(s). "Total solvents" is used to mean the total amount of all such substances. Generally, water in a liquid sugar solution is not included in the calculation of "total solvents". Examples of solvents may be selected from water, propylene glycol, ethanol, polyethylene glycol, glycerol, sorbitol, and mixtures thereof.

In one embodiment, the solvent is selected from water, propylene glycol, ethanol, and mixtures thereof. There are also mixtures of the solvents that may be useful for certain product forms of the present invention. For example, wherein the product form is an elixir, liquid-filled capsule or liquid-filled lozenge, the solvent may optionally be a mixture of propylene glycol, ethanol, and water.

The level of each solvent that makes up the mixture is dependent on the solubility of the active(s) and the aesthetic benefits sought by the formulator. For example, for the compositions of the present invention, the composition may optionally comprise from 30% to 90%, alternatively from 35% to 80%, alternatively from 40% to 70% total solvents, all by weight of the composition. The example ranges of total solvents given above do not include water present in a sugar solution, if a liquid sugar solution is used in the composition.

### Metal Chelators

The present compositions may optionally comprise a metal chelator. It has been found that trace amounts of heavy metal ions may catalyze auto-oxidation reactions that may compromise stability of the final composition.

The compositions may therefore optionally include a chelating agent. Chelating agents are well known to the ordinarily skilled artisan. Examples of chelating agents include disodium and calcium salts of ethylene diamine tetraacetic acid (EDTA), tetrasodium EDTA, sodium hexametaphosphate (SHMP), citric acid, phosphoric acid, di(hydroxyethyl)glycine, 8-hydroxyquinoline, and mixtures thereof. Trivalent metal chelating agents such as galactomannans complexed with iron may also be useful.

Wherein the compositions herein comprise a chelating agent, the compositions may optionally comprise from 0.0001% to 1% of the chelating agent, alternatively from 0.001% to 0.5%, and alternatively from 0.01% to 0.3% of the chelating agent, all by weight of the composition.

### Reducing Agents

The present compositions may also optionally comprise a reducing agent. The inclusion of a reducing agent may have a beneficial chemical stabilizing effect on the pharmaceutical actives used in the present invention. Therefore, the reducing agents useful in the composition depend on the active selected and its solubility.

As used herein, the reducing agent is a substance that has a lower redox potential than the pharmaceutical active or other adjuvant that it is intended to protect from oxidation. Thus, reducing agents are more readily oxidized than the pharmaceutical active or other adjuvant and are effective in the presence of oxidizing agents.

Reducing agents have an "electrode potential value". The electrode potential value is defined by the Nernst equation and measured using standard electrochemical reference cells. The resulting values are therefore called the "Standard Electrode Potential", or E⁰, as measured in volts (V). Comparing Standard Electrode Potentials for different substances can be used to assess the effectiveness of different reducing agents.

The reducing agents useful in the present invention may optionally have E⁰ values greater than about -0.119V, and alternatively from about -0.119V to +0.250V. Illustrative reducing agents are selected from the salts of metabisulfite and bisulfite, including their sodium and potassium salts, dithiothreitol, thiourea, sodium thiosulphate, thioglycolic acid, tert-butyl hydroquinone (TBHQ), acetyl cysteine, hydroquinone, salts thereof, and mixtures thereof.

Wherein a reducing agent is utilized, the present compositions may comprise from 0.001% to 1%, alternatively from 0.01% to 0.5%, and alternatively from 0.05% to 0.1% of a reducing agent, all by weight of the composition.

### Salts

The present compositions may optionally comprise a salt, such as a chloride salt, which has been further discovered to provide potential stability benefits. Examples include sodium chloride, potassium chloride, ammonium chloride, and mixtures thereof.

Wherein the composition comprises a salt, the composition may optionally comprise from 0.0001% to 2%, alternatively from 0.25% to about 1% of the salt, all by weight of the composition. Such salts may slow the dissociation of a pharmaceutical active from the hydrochloride salt of a pharmaceutical active. For example, having a chloride salt present slows the dissociation of phenylephrine from phenylephrine hydrochloride.

### Methods of the Present Invention

In a further embodiment, the present invention is directed to methods of treating a respiratory illness comprising orally administering a composition as described herein to a mammal in need of such treatment. As used herein, the term "respiratory illness" encompasses a broad range of respiratory ailments, including viral infections such as influenza and common cold, as well as allergy, sinusitis, rhinitis, and the like. As further used herein, "treatment" with respect to respiratory illness means that administration of the referenced composition prevents, alleviates, ameliorates, inhibits, or mitigates one or more symptoms of the respiratory illness or the respiratory illness itself, or any like benefit with respect to the respiratory illness in a mammalian subject in need thereof, preferably in humans. As such, this includes, for example: preventing a respiratory illness or its associated symptoms from occurring in a manunal, for example when the mammal is predisposed to acquiring the respiratory illness, but has not yet been diagnosed with the illness; inhibiting the respiratory illness or its associated symptoms: and/or alleviating, reversing, or curing the respiratory illness or its associated symptoms. Insofar as the methods of the present invention are directed to preventing a respiratory illness, it is understood that the term "present" does not require that the respiratory illness be completely thwarted. Rather, as used herein, the term "preventing" or the like refers to the ability of the skilled artisan to identify susceptibility to respiratory illness (such as, for example, in humans during winter months), such that administration of the referenced compositions may occur prior to the onset of the symptoms associated with the illness.

Respiratory illness may present as any of a variety of symptoms, such as runny nose, nasal or chest congestion, cough, sneezing, pressure, headache, aches, fever, or sore throat. The mammal treated may be a human.

As used herein, the term "orally administering" with respect to the mammal means that the mammal ingests or is directed to ingest, or does ingest, one or more of the present compositions. Wherein the human is directed to ingest the composition, such direction may be that which instructs and/or informs the human that use of the composition may and/or will provide the relief from the respiratory illness (e.g. symptomatic relief, whether temporary or permanent) for example, relief from congestion. For example, such direction may be oral direction (e.g., through oral instruction from, or example, a physician, pharmacists, or other heath professional), radio or television media (i.e., advertisement), or written direction (e.g., through written direction from, for example a physician, pharmacist, or other health professional (e.g., scripts), sales professional or organization (e.g., through, for example, marketing brochures, pamphlets, or other instructive paraphernalia), written media (e.g., internet, electronic mail, or other computer-related media), and/or packaging associated with the composition (e.g., a label present on a container holding the composition). As used herein, "written" means through words, pictures, symbols, and/or other visible or tactile descriptors, such as Braille. Such information need not utilize the actual words used herein, for example, "respiratory", "illness", or "mammal", but rather use of words, pictures, symbols and the like conveying the same or similar meaning are contemplated within the scope of this invention.

Administration may be on an as-needed or as-desired basis, for example, once-monthly, once-weekly, or daily, including multiple times daily, for example, at least once daily, twice daily, three times daily, or four times daily or more.

The amount of composition administered may be dependent on a variety of factors, including the general quality of health of the mammal, type of mammal, age, gender, or severity of symptoms. In one embodiment herein, the liquid oral composition is administered to the mammal in total dosage volumes, per dose, of from 5 mL. to 50 mL of the liquid oral composition, alternatively of from 10 mL to 30 mL of the liquid oral composition.

### EXAMPLES

The following examples further describe and demonstrate embodiments within the scope of the present invention.

The compositions below may be made by the following example method. First, propylene glycol and/or polyethylene glycol and/or sorbitol are added to a clean vessel. The acetaminophen, and any optional additional pharmaceutical active(s) such as dextromethorphan, and flavor are added and stirred until dissolved. In a separate vessel, water is added to dissolve phenylephrine, color, buffering agents, and sweeteners, followed by addition of glycerin. The aqueous solution is added to the propylene glycol /polyethylene glycol / sorbitol solution. The resulting solution may be mixed with liquid sugar and additional water to bring (i.e. q.s.) volume to 100%, and the composition is mixed until homogeneous.

### Example 1

Below are illustrated various examples of compositions of the present invention. All examples below would have a pH of about 7.1.

| **Material Description** | **%w/w** | **%w/w** | **%w/w** | **%w/w** | **%w/w** |
|---|---|---|---|---|---|
| ***Glycol Premix*** | | | | | |
| Proplyene Glycol USP | 16 | 16 | 12 | 8 | 16 |
| Polyethylene Glycol 400 NF | 0 | 4 | 8 | 8 | 8 |
| Sorbitol | 8 | 4 | 4 | 8 | 0 |
| Acetaminophen USP | 1.78 | 1.78 | 1.78 | 1.78 | 1.78 |
| Dextromethorphan HBr USP | 0.05 | 0.05 | 0.05 | 0.05 | 0.05 |
| Flavor | 0.30 | 0.30 | 0.30 | 0.30 | 0.31 |

| ***Water Premix*** | | | | | |
|---|---|---|---|---|---|
| Purified Water USP | 6.6 | 4 | 8 | 8 | 6.6 |
| Sodium Citrate Hydrous USP | 1.6 | 1.6 | 1.6 | 1.6 | 1.6 |
| Phenylephrine | 0.033 | 0.033 | 0.033 | 0.033 | 0.033 |
| Citric Acid Anhydrous USP | 0.02 | 0.02 | 0.02 | 0.02 | 0.02 |
| Saccharin Sodium USP | 0.08 | 0.08 | 0.08 | 0.08 | 0.08 |
| Color | 0.06 | 0.06 | 0.06 | 0.06 | 0.06 |
| Glycerin USP | 4 | 8 | 16 | 12 | 4.1 |
| Liquid Sugar # 1 | 53.8 | 49 | 40 | 45 | 53.8 |
| Purified Water USP | QS | QS | QS | QS | QS |
| Propylene Glycol available from Dow Chemical Corp. Plagremine, LA, USA | | | | | |
| Dextromethorphan HBr available from Hoffman-LaRoche, Branchburg, NJ, USA | | | | | |
| Acetaminophen available from Mallinckrodt, Raleigh, NC, USA | | | | | |
| Glycerin available from the Procter & Gamble Company. Cincinnati, OH, USA | | | | | |
| Sodium Citrate available from Hoffman-LaRoche, Branchburg, NJ, USA | | | | | |
| Citric Acid available from ADM, Cork, Ireland | | | | | |
| Phenylephrine HCL available from Iwaki, Ku Tokyo, Japan | | | | | |
| Sodium Saccharin available from PMC Specialties Group, Inc., Cincinnati, OH, USA | | | | | |
| Liquid Sugar available from Imperial Sugar. Port Wentworth, CA, USA | | | | | |

## Claims

1. A liquid composition comprising a pharmaceutical active selected from phenylephrine, its free and addition salt forms, and mixtures thereof; and acetaminophen, the composition being **characterized by** a pH of from 6.75 to 7.5.

2. The composition according to Claim 1 wherein the composition comprises less than 0.1 % by weight of total aldehydes.

3. The composition according to Claim 1 or Claim 2 comprising an additional pharmaceutical active selected from antitussives, antihistamines, non-sedating antihistamines, decongestants, expectorants, analgesics, antipyretic anti-inflammatory agents, local anesthetics, anti-inflammatory agents, demulcents, and mixtures thereof.

4. The composition according to Claim 3 wherein the additional pharmaceutical active is selected from pharmaceutically acceptable forms of dextromethorphan, ephedrine, pseudoephedrine, phenylpropanolamine, ibuprofen, aspirin, ketoprofen, guaifenesin, ambroxyl, bromhexine, diphenhydramine, chlorpheniramine, doxylamine, triprolidine, clemastine, pyrilamine, promethazine, cetirizine, loratidine, oxycodone, hydrocodone, naproxen, brompheniramine, carbinoxamine, caffeine, benzonatate, pheniramine, fentanyl, azatedine, desloratadine, carbamazepine, buprenorphine, hydromorphone, indomethacin, oxymorphone, phenol, codeine, mesalamine, dichlophenac, sulindac, beclomethaxone, meloxicam, fenoproten, mometasone, menthol, benzocaine, dipyridamole, methscopolamine, and mixtures thereof.

5. The composition according to any of the preceding claims further comprising 0.0001% to 1%, preferably 0.01% to 0.3% of a chelating agent selected from ethylene diamine tetraacetic acid (EDTA), disodium and calcium salts of EDTA, tetrasodium EDTA, sodium hexametaphosphate (SHMP), citric acid, phosphoric acid, di(hydroxyethyl)glycine, 8-hydroxyquinoline, salts thereof and mixtures thereof.

6. The composition according to any of the preceding claims further comprising at least one solvent selected from water, propylene glycol, ethanol, polyethylene glycol, glycerol, sorbitol, and mixtures thereof; wherein the composition comprises from 30% to 90%, preferably from 35% to 80%, more preferably from 40% to 70% by weight of total solvents.

7. The composition according to any of the preceding claims further comprising a reducing agent selected from metabisulfite and bisulfite, dithiothritol, thiourea, sodium thiosulphate, thioglycolic acid, tert-butyl hydroquinone, acetyl cysteine, hydroquinone, salts thereof and mixtures thereof.

8. The composition according to any of the preceding claims further comprising from 0.025% to 5% of non-aldehydic flavors by weight of said composition.

9. The composition according to any of the preceding claims further comprising a sweetener; wherein said sweetener comprises sucrose or from 0.0001% to 5% by weight of an artificial sweetener selected from sodium saccharin, acesulfame potassium, sucralose, aspartame, monoammonium glycyrrhizinate, neohesperidin dihydrochalcone, thaumatin, neotame, cyclamates, and mixtures thereof.

10. The composition according to any of the preceding claims further comprising from 0.0001% to 2%, preferably from 0.25% to 1% by weight of a salt selected from sodium chloride, potassium chloride, ammonium chloride, and mixtures thereof.

11. The composition according to any of the preceding claims comprising from 0.0001% to 1% phenylephrine and from 0.01% to 10% of acetaminophen by weight of the composition.

12. A composition according to any preceding claim for use in a method of treating a respiratory illness.

## Patentansprüche

1. Flüssige Zusammensetzung, umfassend: einen pharmazeutischen Wirkstoff, der ausgewählt ist aus Phenylephrin, seinen freien und Additionssalzformen und Mischungen davon; und Acetaminophen, wobei die Zusammensetzung **gekennzeichnet ist durch** einen pH-Wert von 6,75 bis 7,5.

2. Zusammensetzung nach Anspruch 1, wobei die Zusammensetzung weniger als 0,1 Gew.-% an Gesamtaldehyden umfasst.

3. Zusammensetzung nach Anspruch 1 oder Anspruch 2, umfassend einen zusätzlichen pharmazeutischen Wirkstoff, ausgewählt aus Antitussiva, Antihistaminen, nicht-sedierenden Antihistaminen, Abschwellmitteln, Schleimlösern, Schmerzmitteln, fiebersenkenden, entzündungshemmenden Mitteln, örtlichen Betäubungsmitteln, entzündungshemmenden Mitteln, Linderungsmitteln und Mischungen davon.

4. Zusammensetzung nach Anspruch 3, wobei der zusätzliche pharmazeutische Wirkstoff ausgewählt ist aus pharmazeutisch unbedenklichen Formen von Dextromethorphan, Ephedrin, Pseudoephedrin, Phenylpropanolamin, Ibuprofen, Aspirin, Ketoprofen, Guaifenesin, Ambroxyl, Bromhexin, Diphenhydramin, Chlorpheniramin, Doxylamin, Triprolidin, Clemastin, Pyrilamin, Promethazin, Cetirizin, Loratidin, Oxycodon, Hydrocodon, Naproxen, Brompheniramin, Carbinoxamin, Koffein, Benzonatat, Pheniramin, Fentanyl, Azatedin, Desloratadin, Carbamazepin, Buprenorphin, Hydromorphon, Indomethacin, Oxymorphon, Phenol, Codein, Mesalamin, Dichlophenac, Sulindac, Beclomethaxon, Meloxicam, Fenoproten, Mometason, Menthol, Benzocain, Dipyridamol, Methscopolamin und Mischungen davon.

5. Zusammensetzung nach einem der vorstehenden Ansprüche, ferner 0,0001 % bis 1 %, vorzugsweise 0,01 % bis 0,3 % eines Chelatbildners umfassend, der ausgewählt ist aus Ethylendiamintetraessigsäure (EDTA), Dinatrium- und Calciumsalzen von EDTA, Tetranatrium-EDTA, Natriumhexametaphosphat (SHMP), Citronensäure, Phosphorsäure, Di(hydroxyethyl)glycin, 8-Hydroxychinolin, Salzen davon und Mischungen davon.

6. Zusammensetzung nach einem der vorstehenden Ansprüche, ferner mindestens ein Lösungsmittel umfassend, das ausgewählt ist aus Wasser, Propylenglycol, Ethanol, Polyethylenglycol, Glycerol, Sorbitol und Mischungen davon; wobei die Zusammensetzung, bezogen auf die Lösungsmittel insgesamt, von 30 Gew.-% bis 90 Gew.-%, vorzugsweise von 35 Gew.-% bis 80 Gew.-%, mehr bevorzugt von 40 Gew.-%bis 70 Gew.-% umfasst.

7. Zusammensetzung nach einem der vorstehenden Ansprüche, ferner ein Reduktionsmittel umfassend, das ausgewählt ist aus Metabisulfit und Bisulfit, Dithiothritol, Thioharnstoff, Natriumthiosulphat, Thioglycolsäure, tert-Butylhydrochinon, Acetylcystein, Hydrochinon, Salzen davon und Mischungen davon.

8. Zusammensetzung nach einem der vorstehenden Ansprüche, ferner umfassend von 0,025 Gew.-% bis 5 Gew.-% des nicht-aldehydischen Geschmacksstoffs bezogen auf die Zusammensetzung.

9. Zusammensetzung nach einem der vorstehenden Ansprüche, ferner einen Süßstoff umfassend, wobei der Süßstoff Saccharose oder 0,0001 Gew.-% bis 5 Gew.-% eines künstlichen Süßstoffs, ausgewählt aus Natriumsaccharin, Acesulfamkalium, Sucralose, Aspartam, Monoammoniumglycyrrhizinat, Neohesperidindihydrochalcon, Thaumatin, Neotam, Cyclamaten und Mischungen davon, umfasst.

10. Zusammensetzung nach einem der vorstehenden Ansprüche, ferner von 0,0001 Gew.-% bis 2 Gew.-%, vorzugsweise von 0,25 Gew.-% bis 1 Gew.-% ein Salz umfassend, das ausgewählt ist aus Natriumchlorid, Kaliumchlorid, Ammoniumchlorid und Mischungen davon.

11. Zusammensetzung nach einem der vorstehenden Ansprüche, von 0,0001 Gew.-% bis 1 Gew.-% Phenylephrin und von 0,01 Gew.-% bis 10 Gew. -% Acetaminophen umfassend, jeweils bezogen auf die Zusammensetzung.

12. Zusammensetzung nach einem der vorstehenden Ansprüche zur Verwendung in einem Verfahren zur Behandlung einer Atemwegserkrankung.

## Revendications

1. Composition liquide comprenant un agent actif pharmaceutique choisi parmi la phényléphrine, ses formes libres et sels d'addition, et leurs mélanges ; et le paracétamol, la composition étant **caractérisée par** un pH allant de 6,75 à 7,5.

2. Composition selon la revendication 1, la composition comprenant moins de 0,1 % en poids d'aldéhydes totaux.

3. Composition selon la revendication 1 ou la revendication 2 comprenant un agent actif pharmaceutique supplémentaire choisi parmi des antitussifs, antihistaminiques, antihistaminiques sans effet sédatif, décongestionnants, expectorants, analgésiques, agents anti-inflammatoires antipyrétiques, anesthésiques locaux, agents anti-inflammatoires, émollients, et leurs mélanges.

4. Composition selon la revendication 3 dans laquelle ledit agent actif pharmaceutique supplémentaire est choisi parmi des formes pharmaceutiquement acceptables de la dextrométhorphane, de l'éphédrine, de la pseudoéphédrine, de la phénylpropanolamine, de l'ibuprofène, de l'aspirine, du kétoprofène, de la guaïfénésine, de l'ambroxyl, de la bromhexine, de la diphénhydramine, de la chlorphénramine, de la doxylamine, de la triprolidine, de la clémastine, de la pyrilamine, de la prométhazine, de la cétirizine, de la loratadine, de l'oxycodone, de l'hydrocodone, du naproxène, de la bromphéniramine, de la carbinoxamine, de la caféine, du benzonatate, de la phéniramine, du fentanyl, de l'azatadine, de la desloratadine, de la carbamazépine, de la buprénorphine, de l'hydromorphone, de l'indométacine, de l'oxymorphone, du phénol, de la codéine, de la mésalamine, du diclofénac, du sulindac, de la béclométhasone, du méloxicam, du fénoprofène, de la mométasone, du menthol, de la benzocaïne, du dipyridamole, de la methscopolamine et leurs mélanges.

5. Composition selon l'une quelconque des revendications précédentes comprenant en outre de 0,0001 % à 1 %, de préférence de 0,01 % à 0,3 %, d'un agent chélatant choisi parmi l'acide éthylènediaminetétraacétique (EDTA), les sels disodique et de calcium d'EDTA, l'EDTA tétrasodique, l'hexamétaphosphate de sodium (SHMP), l'acide citrique, l'acide phosphorique, la di(hydroxyéthyl)glycine, la 8-hydroxyquinoline, leurs sels et leurs mélanges.

6. Composition selon l'une quelconque des revendications précédentes comprenant, en outre, au moins un solvant choisi parmi l'eau, le propylène glycol, l'éthanol, le polyéthylène glycol, le glycérol, le sorbitol et leurs mélanges ; la composition comprenant de 30 % à 90 %, de préférence de 35 % à 80 %, plus préférablement de 40 % à 70 % en poids du total de solvants.

7. Composition selon l'une quelconque des revendications précédentes comprenant, en outre, un agent réducteur choisi parmi les métabisulfite et bisulfite, dithiothritol, thio-urée, thiosulfate de sodium, acide thioglycolique, tert-butylhydroquinone, acétylcystéine, hydroquinone, leurs sels et leurs mélanges.

8. Composition selon l'une quelconque des revendications précédentes comprenant, en outre, de 0,025 % à 5 % desdits arômes non aldéhydiques en poids de ladite composition.

9. Composition selon l'une quelconque des revendications précédentes comprenant, en outre, un édulcorant ; ledit édulcorant comprenant du sucrose ou de 0,0001 % à 5 % en poids d'un édulcorant de synthèse choisi parmi la saccharine de sodium, l'acésulfame potassium, le sucralose, l'aspartame, le glycyrrhizinate de monoammonium, la néohespéridine dihydrochalcone, la thaumatine, le néotame, les cyclamates et leurs mélanges.

10. Composition selon l'une quelconque des revendications précédentes comprenant, en outre, de 0,0001 % à 2 %, de préférence de 0,25 % à 1 % en poids d'un sel choisi parmi le chlorure de sodium, le chlorure de potassium, le chlorure d'ammonium et leurs mélanges.

11. Composition selon l'une quelconque des revendications précédentes comprenant de 0,0001 % à 1 % de phényléphrine et de 0,01 % à 10 % de paracétamol en poids de la composition.

12. Composition selon l'une quelconque des revendications précédentes, utilisable dans le cadre d'une méthode de traitement d'une maladie respiratoire.
